# EUROPÄISCHE PATENTSCHRIFT

(11) **EP 0 970 028 B1**
(45) Veröffentlichungstag und Bekanntmachung des Hinweises auf die Patenterteilung: **23.01.2002**
(21) Anmeldenummer: 98912482.1
(22) Anmeldetag: 17.03.1998
(51) Int. Cl.: C07C 2/86, C07C 41/30, C07C 67/343

(54) **VERFAHREN ZUR SYNTHESE VON AROMATISCH SUBSTITUIERTEN OLEFINEN**
METHOD FOR SYNTHESISING AROMATICALLY SUBSTITUTED OLEFINS
PROCEDE DE SYNTHESE D'OLEFINES AROMATIQUEMENT SUBSTITUEES

(30) Priorität: 25.03.1997 DE 19712388
(43) Veröffentlichungstag der Anmeldung: 12.01.2000
(73) Patentinhaber: Studiengesellschaft Kohle mbH, 45470 Mülheim an der Ruhr (DE)
(72) Erfinder: REETZ, Manfred, Theodor, D-45470 Mülheim an der Ruhr (DE); LOHMER, Gunther, D-45470 Mülheim an der Ruhr (DE); SCHWICKARDI, Renate, D-45470 Mülheim an der Ruhr (DE)
(74) Vertreter: von Kreisler, Alek, Dipl.-Chem.
(86) Internationale Anmeldenummer: EP9801532
(87) Internationale Veröffentlichungsnummer: WO9842644

(56) Entgegenhaltungen:
- EP-A- 0 103 544
- EP-A- 0 709 357
- DE-A- 19 506 442
- US-A- 4 879 426

## Beschreibung

Die vorliegende Erfindung betrifft ein neues Verfahren zur Synthese von Olefinen mit aromatischen Substituenten mit einem neuen und besonders aktiven Palladiumhaltigen Katalysatorsystem, ggf. in Gegenwart von selektivitätssteigernden Additiven.

In der industriellen Chemie spielen Olefine mit aromatischen Substituenten eine bedeutsame Rolle, so z.B. als Ausgangsprodukte für Polymere, Sonnenschutzmittel (UV-Absorber), Feinchemikalien und Wirkstoffvorprodukte z. B. Pharmaca).

Eine bekannte Methode zur Herstellung von solchen Olefinen ist die sogenannte Heck-Reaktion, bei der Iod- oder Bromaromaten ArX (X = I, Br) und in seltenen Fällen Chloraromaten (X = Cl) mit Olefinen in Gegenwart von stöchiometrischen Mengen einer Base und katalytischen Mengen einer Palladium-Verbindung umgesetzt werden. (F. Heck, "Vinyl Substitutions with Organopalladium Intermediates" in *Comprehensive Organic Syntheses,* Bd. 4, Pergamon Press, Oxford, 1991, S. 833; R. F. Heck, *Palladium Reagents in Organic Syntheses,* Academic Press, London, 1985; R. F. Heck, *Org. React. (N. Y.*) **1982,** 27, 345; A. de Meijere, F. E. Meier, *Angew. Chem*. **1994,** *106*, 2473; J. Tsuji, *Palladium Reagents and Catalysts: Innovations in Organic Synthesis,* Wiley, Chichester, 1995.). 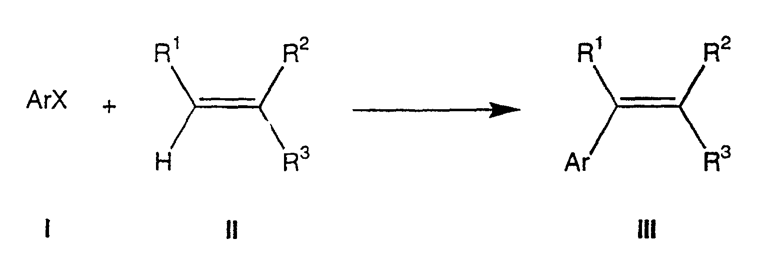 Allerdings hat die Heck-Reaktion bislang keine Bedeutung in der industriellen Anwendung gefunden (B. Cornils, W. A. Herrmann, *Applied Homogeneous Catalysis with Organometallic Compounds,* VCH, Weinheim, 1996). Dies hängt u. a. damit zusammen, daß die Reaktivität von Halogenaromaten ArX sehr stark in der Reihenfolge ArI > ArBr > ArCl abfällt. So werden im universitären Bereich in der Regel die reaktiven lodaromaten eingesetzt, die jedoch für eine industrielle Anwendung viel zu teuer bzw. schwer zugänglich sind. Die gängigen Katalysatoren bzw. Präkatalysatoren wie Palladium-tetrakis(triphenylphosphan) Pd(PPh₃)₄ bzw. Pd(OAc)₂ in Gegenwart von überschüssigem PPh₃ führen im Falle von Bromaromaten zu deutlich schlechteren Ausbeuten, während die Reaktionen der in der Industrie in besonders großen Mengen vorhandenen Chloraromaten mit völlig unbefriedigenden Ausbeuten verlaufen. Als Grund dafür führt Heck die Bildung von Tetraarylphosphonium-Verbindungen an, wodurch der Katalysator unter Abscheidung von elementarem Pd-Pulver zersetzt wird (R. F. Heck, *Org. React. (N. Y.)* **1982,** *27*, 345; C. B: Ziegler, R. F. Heck, J. Org. Chem. 1978, 43, 2941). Tatsächlich gelten katalytische C-C-Verknüpfungsprozesse von reaktionsträgen Chloraromaten, insbesondere im Rahmen von Heck-Reaktionen, als besondere Herausforderung (V. V. Grushin, H. Alper, *Chem. Rev.* **1994,** 94, 1047; B. Cornils, W. A. Herrmann, *Applied Homogeneous Catalysis with Organometallic Compounds,* VCH, Weinheim, 1996).

Die Heck-Reaktion von Chlorbenzol mit Styrol unter Bildung von *trans*-Stilben erfolgt zu 60 %, wenn 1 mol-% Pd(OAc)₂ in Gegenwart von 2 mol-% des Liganden 1,4-Bis(diisopropylphosphino)butan eingesetzt werden. (Y. Ben-David, M. Portnoy, M. Gozin, D. Milstein, *Organometallics* **1992,** *11*, 1995) Dies gehört zu den besten Ergebnissen in der Literatur, wurde jedoch nicht auf elektronenarme Olefine wie Acrylate übertragen, weil das stark nukleophile Phosphan unerwünschte Polymerisation auslöst. Nachteilig ist insbesondere auch die Tatsache, daß relativ große Mengen eines teuren bzw. schwer zugänglichen und oxidationsempfindlichen Liganden erforderlich sind. Ferner gelang die Übertragung auf andere Substrate nur in Einzelfällen. So verläuft z. B. die Reaktion von Styrol mit 4-Chlortoluol nur zu 50 % (Y. Ben-David, M. Portnoy, M. Gozin, D. Milstein, *Organometallics* **1992,** *11*, 1995).

Die Verwendung von Pd-Salzen wie Pd(OAc)₂ in Gegenwart eines Überschusses von Tris(*o*-tolyl)phosphan [P(*o*-Tol)₃] beinhaltet ein aktives Katalysatorsystem, mit dem die Heck-Reaktion von Bromaromaten, insbesondere wenn sie durch elektronenziehende Substituenten aktiviert sind, mit wenig befriedigenden bis guten Ausbeuten (20 - 90 %) verläuft (A. Spencer, *J. Organomet. Chem*. **1983,** *258,* 101; *J. Organomet. Chem.* **1984,** *270*, 115; EP 0078768 A1 und EP 0103544 A1).

Dagegen reagieren aktivierte Chloraromaten mit ausgesprochen schlechten Ausbeuten. Nachteilig in jedem Fall ist die Tatsache, daß P(*o*-Tol)₃ ein teures und schwer zugängliches Phosphan ist.

In neueren Arbeiten wurde berichtet, daß bestimmte Palladacyclen, dargestellt aus Pd(OAc)₂ und P(*o*-Tol)₃, ungewöhnlich aktive Katalysatoren bei der Heck-Reaktion sind. So konnten sogar nicht-aktivierte Bromaromaten wie Brombenzol oder Bromanisol mit n-Butylacrylat zur Reaktion gebracht werden unter Bildung der entsprechenden Heck-Produkte (94 - 96 %) (W. A. Herrmann, C. Broßmer, K. Öfele, C.-P. Reisinger, T. Priermeier, M. Beller, H. Fischer, *Angew. Chem*. **1995,** *107,* 1989; DE 4421730 Cl und EP 0725049 A1). Allerdings gelang die Übertragung auf Chloraromaten nur bedingt. Lediglich aktivierte Chloraromaten wie 4-Chlorbenzaldehyd konnten mit n-Butylacrylat zur Reaktion gebracht werden (81 %) und dies nur in Gegenwart eines zehnfachen Überschusses an Tetrabutylammoniumbromid als Additiv. Nicht-aktivierte Chloraromaten wie Chlorbenzol, 4-Chloranisol oder Chlortoluol konnten nicht umgesetzt werden. Nachteilig bei all diesen Reaktionen ist auch die Tatsache, daß das teure und schwer zugängliche Tris(*o*-tolyl)phosphan bei der Katalysatorherstellung eingesetzt werden muß.

Es hat auch nicht an Versuchen gefehlt, Pd-haltige heterogene Katalysatoren bei der Heck-Reaktion einzusetzen. Während die Ergebnisse bei der Verwendung von Iodaromaten durchaus akzeptabel sind, gibt es bis heute keine allgemein befriedigende Lösung des Problems im Falle von Brom- oder Chloraromaten (V. V. Grushin, A. Alper, *Chem. Rev*. **1994,** *94*, 1047). So werden z.B. bei der Reaktion von Chlorbenzol mit Styrol unter Verwendung von verschiedenen geträgerten Pd-Katalysatoren mäßige Ausbeuten erhalten, auch wenn ein 10-facher Überschuß an Chlorbenzol eingesetzt wird (M. Julia, M. Duteil, C. Grand, E. Kuntz, *Bull. Soc. Chim. Fr*. **1973,** 2791; K. Kaneda, M. Higuchi, T. Imanaka, *J. Mol. Catal*. **1990,** *63,* L33). Unerwünschte Nebenprodukte sind u. a. Benzol und Diphenyl.

EP-A-0 103 544 beschreibt Verfahren zur Pd-katalysierten Arylierung von Olefinen mit Arylchloriden. Es werden zwar quartäre Ammonium-Salze als mögliche Kationen der einzusetzenden Base erwähnt, in den Beispielen wird aber fast ausschließlich Natriumacetat als Base verwendet und quarternäre Ammoniumsalze werden in keinem einzigen Beispiel eingesetzt.

US-A-4 879 426 beschreibt Heck-Reaktionen zur Arylierung von Cycloalkenen in Gegenwart von z.B. Tetra-n-butyl-ammoniumchlorid.

DE-A-195 06 442 beschreibt ein verbessertes Verfahren zur Herstellung von 4-Arylcyclohex-1-enen durch Umsetzung eines Arylhalogenids mit Cyclohexen in Gegenwart von Palladium(II)acetat und einer Base, ausgewählt aus Lithium- oder Natriumacetat. Es werden auch Phasentransferkatalysatoren erwähnt, z.B. Tetraalkylammonium-Salze.

Somit wird klar, daß es nach wie vor einen akuten Bedarf an einfachen bzw. leicht zugänglichen Palladium-Katalysatoren für die Heck-Reaktion von Chlor- und Bromaromaten gibt.

Die vorliegende Erfindung beinhaltet eine Lösung der oben beschriebenen Probleme, da überraschenderweise gefunden wurde, daß Verbindungen des Typs der vorgenannten Formel III mit Hilfe einer Heck-Reaktion leicht zugänglich sind. Als Katalysatoren dienen gängige Palladium-II-Salze PdXY oder deren CH₃CN-, PhCN- oder PPh₃-Komplexe, wobei typischerweise X = Y = Cl, Br, I, RCO₂ [R = C₁ - C₂₂, CF₃, CCl₃, CH₂N(CH₃)₂, C₆H₅] oder RSO₃ (R = C₁ - C₂₂, CF₃, C₄F₉, CCl₃, C₆H₅, *p*-CH₃C₆H₄) oder typischerweise X = Cl, Br, I, RCO₂ (R = C₁ - C₂₂, CF₃, CCl₃, CH₂OCH₃, C₆H₅) und typischerweise Y = C₆H₅, *o-, m-, p*-CH₃C₆H₄, *o-, m-, p*-Cl-C₆H₄, *o-, m-, p*-CHOC₆H₄, *o-, m-, p*-CN-C₆H₄, *o-, m-, p*-NO₂-C₆H₄, *o-, m-, p*-PhCO-C₆H₄, *o-, m-, p*-F-C₆H₄, 1-C₁₀H₇ oder 2-C₁₀H₇, die mit Tetraarylphosphonium-Salzen Ar¹Ar²Ar³Ar⁴P⁺Z⁻, worin Ar¹, Ar², Ar³ und Ar⁴ gleiche oder unterschiedliche Arylreste bedeuten, typischerweise Ar = C₆H₅, *o-, m-, p*-CH₃-C₆H₄, *o-, m-, p*-Cl-C₆H₄, *o-, m-, p*-CHO-C₆H₄, *o-, m-, p*-CN-C₆H₄, *o-, m-, p*-NO₂-C₆H₄, *o-, m-, p*-PhCO-C₆H₄, *o-, m-, p*-F-C₆H₄, 1-C₁₀H₇ oder 2-C₁₀H₇) und Z = Cl, Br, RCO₂ (R = C₁ - C₂₂, CF₃, CCl₃, C₆H₅) oder RSO₃ (R = Cl - C₂₂, CF₃, C₄F₉, C₆H₅, *p*-CH₃C₆H₄) vermischt werden. Vorzugsweise wird PdCl₂, PdCl₂(CH₃CN)₂, Pd(OAc)₂, C₆H₅PdCl oder C₆H₅PdCl·PPh₃ bzw. deren dimere oder oligomere Form in Gegenwart von Tetraphenylphosphoniumchlorid oder -bromid verwendet. Das Verhältnis von PdXY zu Ar¹Ar²Ar³Ar⁴P⁺Z⁻ bewegt sich zwischen 1 : 1 und 1 : 10, vorzugsweise wird ein Verhältnis von 1: 6 gewählt.

Als Lösungsmittel dienen aprotische dipolare Solventien wie Dimethylformamid (DMF), Dimethylacetamid (DMA), Dimethylsulfoxid, Propylencarbonat, 1,3-Dimethyl-3,4,5,6-tetrahydro-2( 1H)-pyrimidinon (DMPU) oder 1-Methyl-2-pyrrolidinon (NMP), vorzugsweise DMF oder NMP.

Als Base dienen Metallsalze wie Natrium-, Kalium-, Cesium-, Calcium- oder Magnesiumsalze von Carbonsäuren oder die entsprechenden Carbonate bzw. Bicarbonate oder Amine wie Triethylamin oder Trioctylamin, vorzugsweise Natriumacetat. Das Verhältnis von Base zu Arylhalogenid bewegt sich zwischen 1 : 1 und 5:1, vorzugsweise 1.5 : 1 bis 2 : 1.

Als selektivitätssteigernde Additive werden stickstoffhaltige Carbonsäuren wie gängige a- oder b-Aminosäuren H₂N(R)CHCO₂H bzw. H₂N(R)CHCH₂CO₂H [R = H, CH₃, C₆H₅, CH₂C₆H₄, CH(CH₃)₂] bzw. deren N-alkylierte Formen R'NH(R)CHCO₂H bzw. R'NH(R)CHCH₂CO₂H oder R'₂N(R)CHCO₂H bzw. R'₂N(R)CHCH₂CO₂H [R' = CH₃, C₂H₅, C₃H₇, C₄H₉ oder R' + R' = (CH2)4 oder (CH₂)₅] bzw. deren Natrium- oder Kaliumsalze, Anthranilsäure bzw. N,N-Dimethylanthranilsäure oder Pyridincarbonsäuren (bzw. deren Natrium- oder Kaliumsalze) wie 2-Pyridincarbonsäure oder aromatische stickstoffhaltige Heterocyclen wie Pyridin, Lutidin, 2,2'-Dipyridyl oder Chinolin verwendet. Vorzugsweise wird N,N-Dimethylglycin eingesetzt. Das Verhältnis von Additiv zu Palladium bewegt sich zwischen 100 : 1 und 1 : 1, vorzugsweise zwischen 50 : 1 und 1 : 1. Die Verwendung von diesen Additiven führt dazu, daß Nebenreaktionen unter unerwünschter Bildung von Reduktions- oder Kupplungsprodukten wie Benzol oder Diphenyl (jeweils aus PhX), die üblicherweise bei der Heck-Reaktion auftreten, oder unerwünschte zweifache Heck-Reaktionen am Olefin, weitgehend oder vollständig unterdrückt werden. Ferner wird die Selektivität zum Trans-Isomeren erhöht. Die stickstoffhaltigen Verbindungen können auch direkt als Additiv und gleichzeitg als Base eingesetzt werden.

Reaktionstemperaturen zwischen 60°C und 180°C können gewählt werden, vorzugsweise läßt man die Reaktionen zwischen 100 °C und 150 °C ablaufen.

Was die Arylkomponente ArX angeht, so können recht unterschiedliche Aryl- und Heteroarylchloride, -bromide, -*o*-tosylate, -*o*-mesylate oder -*o*-triflate eingesetzt werden, beispielsweise Benzol-, Naphthalin-, Pyridin- oder Chinolinderivate.

Bei der Olefinkomponente der vorgenannten Formel **II** stellen R¹, R² und R³ unabhängig voneinander Wasserstoff, Alkyl-(C₁- C₈), Phenyl, 1- oder 2-Naphthyl, Vinyl, O-Alkyl-(C₁ - C₈), O-Phenyl, CN, CO₂H, CO₂-Alkyl-(C₁- C₈), CO₂-Phenyl, CONH₂, CONH-Alkyl-(C₁ - C₅), CON(Alkyl)₂-(C₁ - C₅), Fluor, Chlor, PO(Phenyl)₂, PO(Alkyl)₂-(C₁ - C₅), CO-Phenyl, CO-Alkyl-(C₁ - C₅), NH-Alkyl-(C₁ - C₄), SO₃H, PO₃H, SO₃-Alkyl-(C₁ - C₄) oder SO₂-Alkyl-(C₁ - C4), ferner kommen cyclische Derivate in Frage, nämlich wenn R¹ + R² = (CH₂)ₙ oder R² + R³ = (CH2)n, wobei n = 2 bis 16, sein kann.

### Beispiele:

Die eingesetzten Palladiumkatalysatoren können vor der eigentlichen Reaktion isoliert synthetisiert oder in situ eingesetzt werden.

### Beispiel A: Darstellung von (Ph₄P)₂PdCl₄.

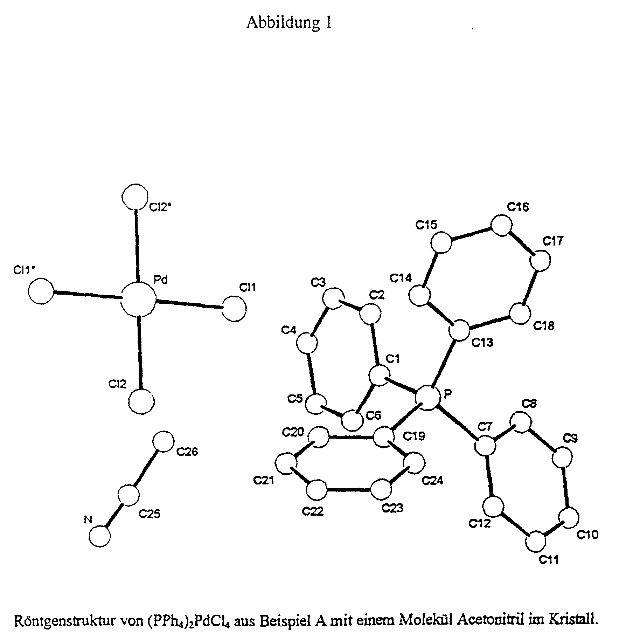

70 mg (0.4 mmol) PdCl₂ und 295.9 mg (0.79 mmol) Ph₄PCl werden in 4 ml Acetonitril wenige Minuten auf 115°C unter Rühren erhitzt. Aus der klaren rot braunen Lösung kristallisieren beim langsamen Abkühlen 350.4 mg (0.38 mmol) rot-braune Nadeln aus. Ausbeute: 95.7 %; Summenformel: C₄₈H₄₀P₂Cl₄Pd , M = 927.02 g/mol
- EA: gef. [%]: 61.64 C, 4.60 H, 6.57 P, 15.83 Cl, 10.84 Pd
ber. [%]: 62.19 C, 4.35 H, 6.68 P, 15.30 Cl. 11.48 Pd.
³¹P-NMR: δ = 23.4 ppm (d₇-DMF)
Röntgenstrukturanalyse: Abbildung 1

### Beispiel B: Darstellung von [PPh₃PdPh(µ-Cl)]₂

112.3 mg (0.50 mmol) Pd(OAc)₂ und 375.3 mg (1.0 mmol) Ph₄PCl werden in 10 ml Acetonitril zu einer rot-braunen Lösung gelöst und 30 Minuten bei RT gerührt. Nach Zugabe von 0.5 ml Ethanol wird im geschlossenen Gefäß 30 Minuten bei 40 °C gerührt. Es fällt sofort ein hellgrüner feiner Niederschlag aus, der beim Erwärmen mehr wird. Der Niederschlag wird isoliert und im Vakuum getrocknet. Ausbeute: 95.2 mg (0.099 mmol, 39.6 %)
Summenformel: C₄₈H₄₀P₂Cl₂Pd₂, M = 962.54 g/mol
- EA: gef. [%]: 59.60 C, 4.14 H, 6.77 P, 7.65 Cl, 22.05 Pd
ber. [%]: 59.89 C, 4.19 H, 6.44 P, 7.37 Cl, 22.11 Pd.

### Beispiel C: [PPh₃PdPh(µ-Cl)]₂+ Na-Dimethylglycinat

48.3 mg (0.05 mmol) [PPh₃PdPh(µ-Cl)]₂ und 13.0 mg (0.10 mmol) Na-Dimethylglycinat werden in 2 ml DMF suspendiert. Beim Erwärmen auf 70 °C entsteht eine klare grüne Lösung.
³¹P-NMR: δ = 31.2 ppm (d₇-DMF)

### Beispiel D: Darstellung von trans-Bis(N,N-Dimethylglycinato)Pd

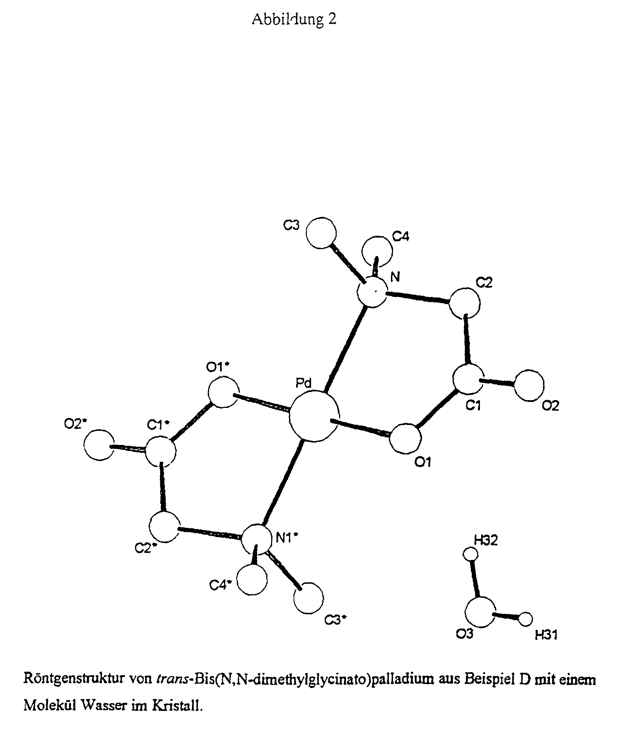

2.0 g (19.4 mmol) Dimethylglycin und 0.58 g (1.97 mmol) Natriumtetrachloropalladat werden bei RT in 10 ml Wasser gelöst. Zu der orange gefärbten Lösung wird 0.473 g (8.43 mmol) KOH gegeben Die hellgelbe Lösung wird nach 3 Stunden Rühren bei RT bis zur Hälfte eingeengt, wobei gelbe Kristalle ausfallen. Diese werden aus heißem Methanol umkristallisiert.
Ausbeute: 0.403 g (1.2 mmol, 60.9 %)
Summenformel: C₈H₁₄N₄O₄Pd, M = 336.64 g/mol
- EA: gef. [%]: 29.10 C, 4.22 H, 16.80 N, 31.3 Pd
ber. [%]: 28.54 C, 4.19 H, 16.64 N, 19.01 O, 31.61 Pd.
Röntgenstrukturanalyse: Abbildung 2

### Beispiel E: (CH₃CN)₂PdCl₂ + 6 Ph₄PCl + 6 Dimethylglycin

25.94 mg (0.1 mmol) (CH₃CN)₂PdCl₂ und 224.89 mg (0.6 mmol) Ph₄PCl werden mit 61.87 mg (0.6 mmol) Dimethylglycin eingewogen und in 5 ml DMF gelöst. Die klare orange Lösung wird 1 Stunde bei RT gerührt, das Lösungsmittel im Vakuum abkondensiert und der Rückstand im Hochvakuum getrocknet. Es verbleiben 304.2 mg orange-brauner Feststoff mit einem Pd-Gehalt von 3.32%.

### Beispiel F: Darstellung der Base Ph₄POAc

In einem 100 ml Rundkolben werden in 20 ml Wasser 0.838g (2.24 mmol) Phosphoniumchlorid und 0.365g (2.19 mmol) Silberacetat gelöst. Nach 1 Stunde wird der voluminöse AgCl-Niederschlag abfiltrtiert und das Wasser wird am Rotationsverdampfer entfernt. Der kristalline Rückstand wird in Ethanol aufgenommen und über Watte filtriert. Das Ethanol wird zur Hälfte entfernt, wobei sich ein weißer kristalliner Feststoff bildet.
Ausbeute: 637.5 mg (1.6 mmol, 73.1 %)
- ¹H-NMR: (200 MHz, d₇-DMF): δ = 1.5 (s) 3 H, CH3-; 7.4 - 7.9 (m) 20 H, Phenyl.
- ¹³C-NMR: (50 MHz, d₇ DMF): δ = 24.5 (s) CH3-; 118.5, 130.9, 135.2, 135.8 (d) Phenyl; 173.0 (s)COO-.

### Beispiele mit Styrol und Br- bzw Cl- Aromaten:

In den folgenden Beispielen wurden die Umsetzungen, sofern nicht anders angegeben, in einem Schlenkgefäß mit Young-Hahn-Verschluß durchgeführt.

### Beispiel 1:

In einem Reaktionsgefäß werden 5.2 mg (0.02 mmol) **(CH**_{**3**}**CN)**_{**2**}**PdCl**_{**2**} und 45.1 mg (0.12 mmol) **Ph**_{**4**}**PCl** eingewogen und anschließend zweimal evakuiert und argoniert. Dazu werden unter Argon 164 mg (2 mmol) wasserfreies **Natriumacetat,** 114.8 mg (1.00 mmol) **Chlorbenzol** und 150.8 mg (1.45 mmol) Styrol zugewogen. Nach Zugabe von 1 ml **NMP** wird das Gefäß verschlossen und das Gemisch zunächst 45 Minuten bei 120 °C und dann 11 Stunden bei 150 °C gerührt.
Nach der Reaktion werden die GC-Standards *n*-Decan und *n*-Hexadecan zugewogen und das Gemisch mit 3 ml Diethylether versetzt. Nach Abfiltrieren der festen Bestandteile wird das Filtrat gaschromatographisch untersucht: Man erhält bei einem 78.5 %igem Cl-Benzol-Umsatz eine 80 %ige Ausbeute an Heck-Produkten (86.5 *% tr*-Stilben, 0.3 % *cis*-Stilben und 13.2 % 1,1-Diphenylethen.

### Beispiel 2:

Es wird wie in Beispiel 1 beschrieben vorgegangen, jedoch als Lösungsmittel **DMF** anstatt NMP verwendet. Es wird 5.5 Stunden bei 150 °C gerührt. Man erhält bei einem 78.9 %igem Cl-Benzol-Umsatz eine 72.1 %ige Ausbeute an Heck-Produkten (86.1 % *tr*-Stilben, 0.9 % *cis*-Stilben, 13.0 % 1,1-Diphenylethen).

### Beispiel 3:

Es wird wie in Beispiel 1 beschrieben vorgegangen, jedoch als Lösungsmittel **DMA** anstatt NMP verwendet. Es wird 8 Stunden bei 150 °C gerührt. Man erhält bei einem 55.2 %igem Cl-Benzol-Umsatz eine 61.2 %ige Ausbeute an Heck-Produkten (83.8 % *tr*-Stilben, 1.1 % *cis*-Stilben, 15.1 % 1,1-Diphenylethen).

### Beispiel 4:

Es wird wie in Beispiel 1 beschrieben vorgegangen, jedoch werden 10.3 mg (0.04 mmol) **(CH**_{**3**}**CN)**_{**2**}**PdCl**_{**2**}**,** 59.7 mg (0.16 mmol) **Ph**_{**4**}**PCl,** 157.6 mg (1.92 mmol) wasserfreies **Natriumacetat,** 107.1 mg (0.95 mmol) **Chlorbenzol** und 136.2 mg (1.31 mmol) **Styrol** in 1 ml **DMF** umgesetzt. Es wird 8 Stunden bei 150 °C gerührt. Man erhält bei einem 95.6 %igem Cl-Benzol-Umsatz eine 70.4 %ige Ausbeute an Heck-Produkten (84.9 % *tr*-Stilben, 0.7 % *cis*-Stilben, 14.4 % 1,1-Diphenylethen).

### Beispiel 5:

Es wird wie in Beispiel 1 beschrieben vorgegangen, jedoch werden 104.2 mg **(1.00 mmol) Styrol** eingesetzt und die Reaktionsmischung wird 8 Stunden bei 150 °C gerührt. Man erhält bei einem 88.7 %igem Cl-Benzol-Umsatz eine 78 %ige Ausbeute an Heck-Produkten (86.5 % *tr*-Stilben, 0.3 % *cis*-Stilben, 13.2 % 1,1-Diphenylethen).

### Beispiel 6:

Es wird wie in Beispiel 1 beschrieben vorgegangen, jedoch werden 102.8 mg **(0.99 mmol) Styrol** und 164.8 mg (1.46 mmol) **Chlorbenzol** eingesetzt und die Reaktionsmischung wird 5.5 Stunden bei 150 °C gerührt. Man erhält bei einem 94.6 %igem Cl-Benzol-Umsatz eine 82.1 %ige Ausbeute an Heck-Produkten (86.7 % *tr*-Stilben, 0.9 % *cis*-Stilben, 12.4 % 1,1-Diphenylethen).

### Beispiel 7:

Es wird wie in Beispiel 1 beschrieben vorgegangen, jedoch werden als Katalysator 13.9 mg (0.02 mmol) **(Ph**_{**3**}**P)**_{**2**}**PdCl**_{**2**} und 29.9 mg (0.08mmol) **Ph**_{**4**}**PCl** in 1 ml **DMF** eingesetzt. Es wird 5.5 Stunden bei 150 °C gerührt. Man erhält bei einem 77.2 %igem Cl-Benzol-Umsatz eine 69.6 %ige Ausbeute an Heck-Produkten (84.7 % *tr*-Stilben, 0.8 % *cis*-Stilben, 14.5 % 1,1-Diphenylethen).

### Beispiel 8:

Es wird wie in Beispiel 1 beschrieben vorgegangen, jedoch werden 18.5 mg (0.02 mmol) **(Ph**_{**4**}**P)**_{**2**}**PdCl**_{**4**} (Darstellung Bsp. A) unter Zusatz von 0.08 mmol **Ph**_{**4**}**PCl** als Katalysator in 1 ml **DMF** verwendet. Es wird 0.3 Stunden bei 120 °C und 12 Stunden bei 150 °C gerührt. Man erhält bei einem 80 %igem Cl-Benzol-Umsatz eine 85.1 %ige Ausbeute an Heck-Produkten (86.1 % *tr*-Stilben, 0.9 % *cis*-Stilben, 13.0 % 1,1-Diphenylethen).

### Beispiel 9:

Es wird wie in Beispiel 1 beschrieben vorgegangen, jedoch werden 9.6 mg (0.02 mmol) **[PPh**_{**3**}**PdPh(µ-Cl)]** (Darstellung Bsp. B) unter Zusatz von 44.98 mg (0.121 mmol) **Ph**_{**4**}**PCl** als Katalysator in 1 ml **NMP** verwendet. Es wird 12 Stunden bei 150 °C gerührt. Man erhält bei einem 86.2 %igem Cl-Benzol-Umsatz eine 86.4 %ige Ausbeute an Heck-Produkten (84.8 % *tr*-Stilben, 0.9 % *cis*-Stilben, 14.4 % 1,1-Diphenylethen).

### Beispiel 10:

Es wird wie in Beispiel 1 beschrieben vorgegangen, jedoch werden als Katalysator 5.6 mg (0.02 mmol) **Pd(tetramethylethylendiamin)Me**_{**2**} und 46.0 mg (0.12 mmol) **Ph**_{**4**}**PCl** in 1 ml **NMP** eingesetzt unter Zusatz von 9.9 mg (0.1 mmol) **Dimethylglycin.** Es wird 12 Stunden bei 150 °C gerührt. Man erhält bei einem 84.9 %igem Cl-Benzol-Umsatz eine 81.5 %ige Ausbeute an Heck-Produkten (96.8 % *tr*-Stilben, 0.7 % *cis*-Stilben, 2.5 % 1,1-Diphenylethen).

### Beispiel 11:

Es wird wie in Beispiel 1 beschrieben vorgegangen, jedoch werden als Katalysator 18.6 mg (0.02 mmol) **Pd(dibenzylidenaceton)**_{**2**} und 45.0 mg (0.12 mmol) **Ph**_{**4**}**PCl** in 1 ml **DMF** eingesetzt. Es wird 6 Stunden bei 150 °C gerührt. Man erhält bei einem 26.5 %igem Cl-Benzol-Umsatz eine 34.2 %ige Ausbeute an Heck-Produkten (84.3 % *tr*-Stilben, 1.4 % *cis*-Stilben, 14.4 % 1,1-Diphenylethen).

### Beispiel 12:

Es wird wie in Beispiel 1 beschrieben vorgegangen, jedoch wird als Base 195.7 mg (2.04 mmol) **Na-propionat** in 1 ml **DMF** verwendet. Es wird 12 Stunden bei 150 °C gerührt. Man erhält bei einem 73 %igem Cl-Benzol-Umsatz eine 55.1 %ige Ausbeute an Heck-Produkten (84.0 % *tr*-Stilben, 1.9 % *cis*-Stilben, 14.9 % 1,1-Diphenylethen).

### Beispiel 13:

Es wird wie in Beispiel 12 beschrieben vorgegangen, jedoch wird als Lösungsmittel 1 ml **NMP** verwendet. Man erhält bei einem 75 %igem Cl-Benzol-Umsatz eine 68.0 %ige Ausbeute an Heck-Produkten (84.2 % *tr*-Stilben, 1.9 % *cis*-Stilben, 14.7 % 1,1-Diphenylethen).

### Beispiel 14:

Es wird wie in Beispiel 1 beschrieben vorgegangen, jedoch wird als Base 144.3 mg (1.0 mmol) **Na-benzoat** in 1 ml **DMF** verwendet. Es wird 5.5 Stunden bei 150 °C gerührt. Man erhält bei einem 52.4 %igem Cl-Benzol-Umsatz eine 55.5 %ige Ausbeute an Heck-Produkten (84.4 % *tr*-Stilben, 1.0 % *cis*-Stilben, 14.7 % 1,1-Diphenylethen).

### Beispiel 15:

Es wird wie in Beispiel 14 beschrieben vorgegangen, jedoch wird als Lösungsmittel 1 ml **NMP** verwendet. Man erhält bei einem 60 %igem Cl-Benzol-Umsatz eine 58.0 %ige Ausbeute an Heck-Produkten (84.2 % *tr*-Stilben, 1.9 % *cis*-Stilben, 14.7% 1,1-Diphenylethen).

### Beispiel 16:

Es wird wie in Beispiel 1 beschrieben vorgegangen, jedoch wird als Base 311.6 mg (2.15 mmol) **Na-picolat** in 1 ml **DMF** verwendet. Es wird 12 Stunden bei 150 °C gerührt. Man erhält bei einem 26.7 %igem Cl-Benzol-Umsatz eine 32.1 %ige Ausbeute an Heck-Produkten (97.4 % *tr*-Stilben, 1.1 % *cis*-Stilben, 1.5 % 1,1-Diphenylethen).

### Beispiel 17:

Es wird wie in Beispiel 16 beschrieben vorgegangen, jedoch wird als Lösungsmittel 1 ml **NMP** verwendet. Man erhält bei einem 31.5 %igem Cl-Benzol-Umsatz eine 34.0 %ige Ausbeute an Heck-Produkten (84.2 % *tr*-Stilben, 1.9 % *cis*-Stilben, 14.7 % 1,1- Diphenylethen).

### Beispiel 18:

Es wird wie in Beispiel 1 beschrieben vorgegangen, jedoch wird als Additiv 12.5 mg (0.12 mmol) **Dimethylglycin** zu den ersten beiden Edukten zugewogen. Es wird 30 Minuten bei 120 °C und 12 Stunden bei 150 °C gerührt. Man erhält bei einem 95.6 %igem Cl-Benzol-Umsatz eine 100 %ige Ausbeute an Heck-Produkten (96.4 % *tr*-Stilben, 0.7 % *cis*-Stilben, 2.9% 1,1-Diphenylethen).

### Beispiel 19:

Es wird wie in Beispiel 18 beschrieben vorgegangen, jedoch wird als Lösungsmittel 1 ml **DMF** verwendet. Man erhält bei einem 95.2 %igem Cl-Benzol-Umsatz eine 83.6 %ige Ausbeute an Heck-Produkten (96.4 % *tr*-Stilben, 0. 7 % *cis*-Stilben, 2.9 % 1,1-Diphenylethen).

### Beispiel 20:

Es wird wie in Beispiel 1 beschrieben vorgegangen, jedoch werden als Katalysator 13.9 mg (0.02 mmol) **(Ph**_{**3**}**P)**_{**2**}**PdCl**_{**2**} und 29.9 mg (0.08 mmol) **Ph**_{**4**}**PCl** in 1 ml **NMP** eingesetzt unter Zusatz von 9.9 mg (0.1 mmol) **Dimethylglycin.** Es wird 12 Stunden bei 150 °C gerührt. Man erhält bei einem 81.4 %igem Cl-Benzol-Umsatz eine 79.6 %ige Ausbeute an Heck-Produkten (96.81 % *tr*-Stilben, 0.71 % *cis*-Stilben, 2.5 % 1,1-Diphenylethen).

### Beispiel 21:

Es wird wie in Beispiel 1 beschrieben vorgegangen, jedoch werden 9.6 mg (0.02 mmol) **[PPh**_{**3**}**PdPh(µ-Cl)]** unter Zusatz von 44.98 mg (0.12 mmol) **Ph**_{**4**}**PCl** als Katalysator in 1 ml **NMP** unter Zusatz von 9.8 mg (0.1 mmol) **Dimethylglycin.** Es wird 12 Stunden bei 150 °C gerührt. Man erhält bei einem 89.2 %igem Cl-Benzol-Umsatz eine 87.4 %ige Ausbeute an Heck-Produkten (96.9 % *tr*-Stilben, 0.9 % *cis*-Stilben, 2.2 % 1,1-Diphenylethen).

### Beispiel 22:

Es wird wie in Beispiel 1 beschrieben vorgegangen, jedoch werden 0.02 mmol der bei **Beispiel C** beschriebenen Katalysatorlösung unter Zusatz von 44.98 mg (0.12 mmol) **Ph**_{**4**}**PCl** als Katalysator in 1 ml **NMP** verwendet. Es wird 12 Stunden bei 150 °C gerührt. Man erhält bei einem 82.4 %igem Cl-Benzol-Umsatz eine 83.5 %ige Ausbeute an Heck-Produkten (96.9 % *tr*-Stilben, 0.9 % *cis*-Stilben, 2.2 % 1,1-Diphenylethen).

### Beispiel 23:

Es wird wie in Beispiel 1 beschrieben vorgegangen, jedoch werden 6.7 mg (0.02 mmol) des bei **Beispiel D** beschriebenen Katalysators unter Zusatz von 45 mg (0.12 mmol) **Ph**_{**4**}**PCl** als Katalysator in 1 ml **NMP** verwendet. Es wird 12 Stunden bei 150 °C gerührt. Man erhält bei einem 91.5 %igem Cl-Benzol-Umsatz eine 89.7 %ige Ausbeute an Heck-Produkten (96.7 % *tr*-Stilben, 0.9 % *cis*-Stilben, 2.4 % 1,1-Diphenylethen).

### Beispiel 24:

Es wird wie in Beispiel 1 beschrieben vorgegangen, jedoch werden 64.1 mg (0.02 mmol) des bei **Beispiel E** beschriebenen Katalysators in 1 ml **NMP** verwendet. Es vird 12 Stunden bei 150 °C gerührt. Man erhält bei einem 98.2 %igem Cl-Benzol-Umsatz eine 97.6 %ige Ausbeute an Heck-Produkten (97.1 % *tr*-Stilben, 0.9 % *cis*-Stilben, 2.0 % 1,1-Diphenylethen).

### Beispiel 25:

Es wird wie in Beispiel 1 beschrieben vorgegangen, jedoch werden 18.5 mg (0.02 mmol) **(Ph**_{**4**}**P)**_{**2**}**PdCl**_{**4**} unter Zusatz von 30.0 mg (0.08 mmol) **Ph**_{**4**}**PCl** als Katalysator in 1 ml **DMF** unter Zusatz von 9.6 mg (0.09 mmol) **Dimethylglycin** verwendet. Es wird 0.3 Stunden bei 120 °C und 12 Stunden bei 150 °C gerührt. Man erhält bei einem 98 %igem Cl-Benzol-Umsatz eine 97.4 %ige Ausbeute an deck-Produkten (96.1 % *tr*-Stilben, 0.9 % *cis*-Stilben, 3.0 % 1,1-Diphenylethen).

### Beispiel 26:

Es wird wie in Beispiel 1 beschrieben vorgegangen, jedoch wird als Additiv 14.8 mg (0.12 mmol) **Picolinsäure** verwendet und nur 12 Stunden bei 150 °C gerührt.

Man erhält bei einem 65.6 %igem Cl-Benzol-Umsatz eine 70.1 %ige Ausbeute an Heck-Produkten (96.9 % *tr*-Stilben, 0.9 % *cis*-Stilben, 2.2 % 1,1-Diphenylethen).

### Beispiel 27:

Es wird wie in Beispiel 26 beschrieben vorgegangen, jedoch wird als Lösungsmittel 1 ml **DMF** verwendet. Man erhält bei einem 89.0 %igem Cl-Benzol-Umsatz eine 81%ige Ausbeute an Heck-Produkten (96.9 % *tr*-Stilben, 0.9 % *cis*-Stilben, 2.2 % 1,1-Diphenylethen).

### Beispiel 28:

Es wird wie in Beispiel 1 beschrieben vorgegangen, jedoch mit 50,3 mg **Ph**_{**4**}**PBr** (0,12 mmol) anstelle von Ph₄PCl und als Lösungsmittel 1 ml DMF. Man erhält bei 74,8 %igem Chlorbenzol-Umsatz eine 77,3 %ige Ausbeute an Heck-Produkten (85,9 % trans-Stilben, 0,6 % cis-Stilben, 13,5 % 1,1-Diphenylethen).

### Beispiel 29:

Es wird wie in Beispiel 1 beschrieben vorgegangen, jedoch wird als Phosphoniumsalz 47.8 mg (0.12 mmol) **Ph**_{**4**}**POAc** in 1 ml **DMF** eingesetzt. Es wird 12 Stunden bei 150 °C gerührt. Man erhält bei einem 73.5 %igem Cl-Benzol-Umsatz eine 71.9 %ige Ausbeute an Heck-Produkten (85.7 % *tr*-Stilben, 0.9 % *cis*-Stilben, 13.4% 1,1-Diphenylethen).

### Beispiel 30:

Es wird wie in Beispiel 1 beschrieben vorgegangen, jedoch wird als Additiv 15.7 mg (0.12 mmol) **Piperidin-2-carbonsäure** verwendet. Man erhält bei einem 84 %igem Cl-Benzol-Umsatz eine 87%ige Ausbeute an Heck-Produkten (96.2 % *tr*-Stilben, 0.8 % *cis*-Stilben, 2.97 % 1,1-Diphenylethen).

### Beispiel 31:

Es wird wie in Beispiel 1 beschrieben vorgegangen, jedoch wird als Additiv 14.1 mg (0.12 mmol) **Prolin** verwendet und nur 12 Stunden bei 150 °C gerührt. Man erhält bei einem 69.4 %igem Cl-Benzol-Umsatz eine 71.1 %ige Ausbeute an Heck-Produkten (94.7 % *tr*-Stilben, 0.1 % *cis*-Stilben, 4.4 % 1,1-Diphenylethen).

### Beispiel 32:

Es wird wie in Beispiel 31 beschrieben vorgegangen, jedoch wird als Lösungsmittel 1 ml **DMF** verwendet. Man erhält bei einem 89.6 %igem Cl-Benzol-Umsatz eine 82.6%ige Ausbeute an Heck-Produkten (92.3 % *tr*-Stilben, 0.8 % *cis*-Stilben, 6.8 % 1,1-Diphenylethen).

### Beispiel 33:

Es wird wie in Beispiel 1 beschrieben vorgegangen, jedoch werden 3.8 mg (0.015 mmol) **(CH**_{**3**}**CN)**_{**2**}**PdCl**_{**2**} und 34.0 mg (0.09 mmol) **Ph**_{**4**}**PCl** als Katalysator und als Additiv 147.1 mg (1.86 mmol) **Pyridin** zur Reaktion gebracht. Es wird 20 Stunden bei 150 °C gerührt. Man erhält bei einem 86.4 %igem Cl-Benzol-Umsatz eine 67.9 %ige Ausbeute an Heck-Produkten (91.6 % *tr*-Stilben, 0.6 % *cis*-Stilben, 7.8 % 1,1-Diphenylethen).

### Beispiel 34:

In einem Reaktionsgefäß, das mit Septumkappe verschlossen wird, werden 5.3 mg (0.02 mmol) **(CH**_{**3**}**CN)**_{**2**}**PdCl**_{**2**}**,** 45 mg (0.12mmol) **Ph**_{**4**}**PCl** und 12.4 mg (0.12 mmol) **Dimethylglycin** eingewogen und anschließend zweimal evakuiert und argoniert. Dazu werden unter Argon 657.7 mg (8 mmol) wasserfreies Natriumacetat zugewogen und 1 ml **NMP** zugegeben. 452 mg (4.01 mmol) **Cl-Benzol** und 636 mg (6.11 mmol) **Styrol** werden mit 0.5 ml **NMP** vermischt und in 6 Portionen innerhalb 6 Stunden zugetropft, die erste Portion bei RT, die weiteren bei 140°C. Insgesamt wird 24 h bei 140 °C gerührt. Nach der bei Beispiel 1 beschriebenen Aufarbeitung unter Verwendung von 10 ml Diethylether erhält man bei einem 76.6 %igern Cl-Benzol-Umsatz eine 64.2 %ige Ausbeute an Heck-Produkten (97.4 % *tr*-Stilben, 0.6 % *cis*-Stilben und 2.0 % 1,1-Diphenylethen).

### Beispiel 35:

Es wird wie in Beispiel 1 beschrieben vorgegangen, jedoch werden 3.9 mg (0.015 mmol) **(CH**_{**3**}**CN)**_{**2**}**PdCl**_{**2**}**,** 34.9 mg (0.09 mmol) **Ph**_{**4**}**PCl,** 520.9 mg (6.35 mmol) wasserfreies **Natriumacetat,** 499.6 mg (3.118 mmol) **Br-Benzol** und 489.3 mg (4.7 mmol) **Styrol** in 1.5 ml **DMF** umgesetzt. Es wird 5 Stunden bei 130 °C gerührt. Nach der bei Beispiel 1 beschriebenen Aufarbeitung erhält man bei einem 74.6 %igem Br-Benzol-Umsatz eine 74.8 %ige Ausbeute an Heck-Produkten (85.0 % *tr*-Stilben, 0.5 % *cis*-Stilben und 14.5 % 1,1-Diphenylethen).

### Beispiel 36:

Es wird wie in Beispiel I beschrieben vorgegangen, jedoch werden 503.5 mg (3.2 mmol) **p-Nitro-chlorbenzol** und 493.9 mg (4.74 mmol) **Styrol** in 1.5 ml **DMF** zur Reaktion gebracht. Als Katalysator wird 3.9 mg (0.015 mmol) **(CH**_{**3**}**CN)**_{**2**}**PdCl**_{**2**}**,** und 34.9 mg (0.093 mmol) **Ph**_{**4**}**PCl,** verwendet. Es wird 12 Stunden bei 135 °C gerührt. Nach der bei Beispiel 1 beschriebenen Aufarbeitung erhält man bei einem 68.5 %igem p-Nitro-chlorbenzol-Umsatz eine 15.9 %ige Ausbeute an Heck-Produkten.

### Beispiel 37:

Es wird wie in Beispiel 1 beschrieben vorgegangen, jedoch werden 124.7 mg (0.99 mmol) **p-Chlor-Toluol** und 113.7 mg (1.09 mmol) **Styrol** in 1ml **DMF** zur Reaktion gebracht. Als Katalysator wird 10.4 mg (0.04 mmol) **(CH**_{**3**}**CN)**_{**2**}**PdCl**_{**2**}**,** und 89.6 mg (0.24 mmol) **Ph**_{**4**}**PCl,** verwendet. Es wird 4.3 Stunden bei 150 °C gerührt. Nach der bei Beispiel 1 beschriebenen Aufarbeitung erhält man bei einem 70.7 %igem p-Chlor-Toluol-Umsatz eine 22.9 %ige Ausbeute an Heck-Produkten.

### Beispiel 38:

Es wird wie in Beispiel 1 beschrieben vorgegangen, jedoch werden 160.1 mg (1.036 mmol) **p-Chlor-Acetophenon** und 125.8 mg (1.21 mmol) **Styrol** in 1ml **NMP** zur Reaktion gebracht. Als Katalysator wird 5.1 mg (0.02 mmol) **(CH**_{**3**}**CN)**_{**2**}**PdCl**_{**2**}**,** und 44.8 mg (0.12 mmol) **Ph**_{**4**}**PCl,** verwendet. Es wird 0.5 Stunden bei 120 °C und 12 Stunden bei 150 °C gerührt. Nach der bei Beispiel 1 beschriebenen Aufarbeitung erhält man bei einem 74.6 %igem p-Chlor-Acetophenon-Umsatz eine 48.2 %ige Ausbeute an Heck-Produkten.

### Beispiel 39:

Es wird wie in Beispiel 1 beschrieben vorgegangen, jedoch werden 3.9 mg (0.015 mmol) **(CH**_{**3**}**CN)**_{**2**}**PdCl**_{**2**}**,** 34.9 mg (0.09 mmol) **Ph**_{**4**}**PCl,** 496.0 mg (6.05 mmol) wasserfreies **Natriumacetat,** 638.74 mg (3.07 mmol) **3-Brom-chinolin** und 427.0 mg (4.1 mmol) **Styrol** in 1.5 ml **DMF** umgesetzt. Es wird 5 Stunden bei 130 °C gerührt. Nach der bei Beispiel 1 beschriebenen Aufarbeitung erhält man bei einem 78.5 %igem 3-Brom-chinolin -Umsatz eine 72.5 %ige Ausbeute an Heck-Produkten (85.1 % *tr*-Stilben, 0.8 % *cis*-Stilben und 14.1 % 1,1-Diphehylethen

### Beispiele mit Acrylsäureestern und Br- bzw Cl-Aromaten:

### Beispiel 40:

In einem Reaktionsgefäß werden 5.1 mg (0.02 mmol) **(CH**_{**3**}**CN)**_{**2**}**PdCl**_{**2**}**,** und 45 mg (0.12mmol) **Ph**_{**4**}**PCl** eingewogen und anschließend zweimal evakuiert und argoniert. Dazu werden unter Argon 147.7 mg (1.8 mmol) wasserfreies **Natriumacetat,** 110.4 mg (0.98 mmol) **Chlorbenzol** und 184.2 mg (1.0 mmol) **Acrylsäureethylhexylester** zugewogen. Nach Zugabe von 1 ml **NMP** wird das Gefäß verschlossen und das Gemisch zunächst 60 Minuten bei 120°C und dann 20 Stunden bei 150 °C gerührt.

Nach der Reaktion werden die GC-Standards *n*-Decan und *n*-Hexadecan zugewogen und das Gemisch mit 3 ml Diethylether versetzt. Nach Abfiltrieren der festen Bestandteile wird das Filtrat gaschromatographisch untersucht: Man erhält bei einem 31.6 %igem Cl-Benzol-Umsatz eine 34.6 %ige Ausbeute an Heck-Produkt.

### Beispiel 41:

Es wird wie in Beispiel 40 beschrieben vorgegangen, jedoch unter Verwendung von 5.0 mg (0.019 mmol) **(CH**_{**3**}**CN)**_{**2**}**PdCl**_{**2**}**,** 45 mg (0.12mmol) **Ph**_{**4**}**PCl,** 177.2 mg (2.16 mmol) **Natriumacetat,** 116.4 mg (1.03 mmol) **Chlorbenzol,** 152.9 mg (1.193 mmol) **Acrylsäurebutylester** und 1 ml **DMF.** Es wird 5.5 Stunden bei 160 °C gerührt. Man erhält bei einem 29.5 %igem Cl-Benzol-Umsatz eine 14.8 % ige Ausbeute an Heck-Produkt.

### Beispiel 42:

Es wird wie in Beispiel 40 beschrieben vorgegangen, jedoch unter Verwendung von 5.3 mg (0.02 mmol) **(CH**_{**3**}**CN)**_{**2**}**PdCl**_{**2**}**,** 45.2 mg (0.12 mmol) **Ph**_{**4**}**PCl,** 185.4 mg (2.26 mmol) **Natriumacetat,** 120.3 mg (1.07 mmol) **Chlorbenzol,** 189.4 mg (1.48 mmol) **Acrylsäure-tert.-butylester** und 1 ml **NMP.** Es wird 1 Stunde bei 120 °C und 20 Stunden bei 150 °C gerührt. Man erhält bei einem 28.7 %igem Cl-Benzol-Umsatz eine 27.5 % ige Ausbeute an Heck-Produkt.

### Beispiel 43:

Es wird wie in Beispiel 40 beschrieben vorgegangen, jedoch unter Verwendung von 5.1 mg (0.02 mmol) **(CH**_{**3**}**CN)**_{**2**}**PdCl**_{**2**}**,** 45 mg (0.12mmol) **Ph**_{**4**}**PCl,** 228 mg (2.8 mmol) Natriumacetat, 120.0 mg (1.07 mmol) **Chlorbenzol,** 337.8 mg (1.70 mmol) **Methacrylsäureethylhexylester** und **1** ml DMF. Man erhält bei einem 93 %igem Cl-Benzol-Umsatz eine ca. 40 % ige Ausbeute an Heck-Produkt.

### Beispiel 44:

Es wird wie in Beispiel 40 beschrieben vorgegangen, jedoch unter Verwendung von 1.3 mg (0.005 mmol) **(CH**_{**3**}**CN)**_{**2**}**PdCl**_{**2**}**,** 11.9 mg (0.032 mmol) **Ph**_{**4**}**PCl,** 1.676 g (20.43 mmol) **Natriumacetat,** 1.54g (9.81 mmol) **Br-Benzol,** 1.86g (14.48 mmol) **Acrylsäurebutylester** und 5 ml **DMF.** Bei der Aufarbeitung werden 10 ml Diethylether verwendet. Man erhält bei einem 100 %igem Br-Benzol-Umsatz eine 92.3 % ige Ausbeute an Heck-Produkt.

### Beispiel 45:

Es wird wie in Beispiel 40 beschrieben vorgegangen, jedoch unter Verwendung von 5.4 mg (0.02 mmol) **(CH**_{**3**}**CN)**_{**2**}**PdCl**_{**2**}**,** 45 **mg** (0.12mmol) **Ph**_{**4**}**PCl,** 175 mg (2.13 mmol) **Natriumacetat,** 196.9 mg (1.05 mmol) **p-Br-Anisol,** 328.9 mg (1.79 mmol) **Acrylsäureethylhexylester** und 1 ml **NMP.** Man erhält bei einem 100 %igem p-Br-Anisol-Umsatz eine. 90 % ige Ausbeute an Heck-Produkt.

### Beispiele mit Cyclohexen und Halogenaromaten

### Beispiel 46:

Es wird wie in Beispiel 1 beschrieben vorgegangen, jedoch 162.6 mg (1.04 mmol) **Brombenzol** und 121.9 mg (1.48 mmol) **Cyclohexen** mit gleichem Katalysator unter Zusatz von 14.1 mg (0.14 mmol) **Dimethylglycin** als Additiv in 1 ml **NMP** umgesetzt. Es wird 8 Stunden bei 140 °C gerührt. Man erhält bei einem 38.5 %igem Brombenzol-Umsatz eine 30.9 %ige Ausbeute an Heck-Produkten (Doppelbindungsisomerenverhältnis 48.4: 51.6).

### Beispiel 47:

Es wird wie in Beispiel 1 beschrieben vorgegangen, jedoch 115.9 mg (1.03 mmol) **Chlorbenzol** und 120.0 mg (1.4 mmol) **Cyclohexen** mit gleichem Katalysator unter Zusatz von 13.0 mg (0.13 mmol) **Dimethylglycin** in 1 ml **NMP** umgesetzt. Es wird 12 Stunden bei 150 °C gerührt. Man erhält bei einem 22.3 %igem Chlorbenzol-Umsatz eine 17.7 %ige Ausbeute an Heck-Produkten (Doppelbindungsisomerenverhältnis 28.4: 71.6).

### Beispiel 48:

Es wird wie in Beispiel 1 beschrieben vorgegangen, jedoch 212.1 mg (1.07 mmol) **Bromacetophenon** und 122.7 mg (1.49 mmol) **Cyclohexen** mit gleichem Katalysator unter Zusatz von 14.7 mg (0.14 mmol) **Dimethylglycin** als Additiv in 1 ml **NMP** umgesetzt. Es wird 8 Stunden bei 140 °C gerührt. Man erhält bei einem ca. 10 %igem Bromacetophenon-Umsatz eine 10.3 %ige Ausbeute an Heck-Produkten (Doppelbindungsisomerenverhältnis 43.7 : 56.3).

### Beispiel mit Ethylen und Halogenaromaten

### Beispiel 49:

11.85 g (50 mmol) **2-Brom-6-methoxynaphthalin** werden in 50 ml **DMF** gelöst und mit 8.2 g (100 mmol) **NaOAc,** 260 mg (1 mmol) **(CH**_{**3**}**CN)**_{**2**}**PdCl**_{**2**} und 225.1 mg (6 mmol) **Ph**_{**4**}**PCl** in einen 100 ml VA-Stahlautoklav mit Magnetrührung überführt. Es wird 14 Stunden bei einem Ethylendruck von 20 bar gerührt. Ausbeute an 2-Vinyl-6-methoxynaphthalin: 78 %.

## Patentansprüche

1. Verfahren zur Herstellung von olefinierten Aromaten oder Heteroaromaten der Formel **III,** 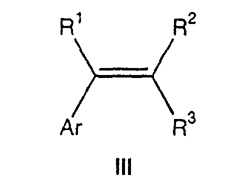 worin Ar substituierte oder nicht-substituierte Aryl- oder Heteroaryl-Reste darstellen, und R¹, R² und R³ unabhängig voneinander Wasserstoff, Alkyl-(C₁ - C₈), Phenyl, 1- oder 2-Naphthyl, Vinyl, O-Alkyl-(C₁ - C₈), O-Phenyl, CN, CO₂H, CO₂-Alkyl-(C₁ - C₈), CO₂-Phenyl, CONH₂, CONH-Alkyl-(C₁ - C₅), CON(Alkyl)₂-(C₁- C5), Fluor, Chlor, PO(Phenyl)₂, PO(Alkyl)₂-(C₁ - C₅), CO-Phenyl, CO-Alkyl-(C₁ - C₅), NH-Alkyl-(C₁ - C₄), SO₃H, PO₃H, SO₃-Alkyl-(C₁ - C₄) oder SO₂-Alkyl-(C₁ - C₄) oder R¹ + R² = (CH₂)ₙ oder R² + R³ = (CH₂)ₙ - n = 2-16 - bedeuten, durch Umsetzung von Aromaten oder Heteroaromaten der Formel **I,**
ArX **I**
worin Ar die gleiche Bedeutung hat wie bei Formel **III,** und X Chlor, Brom, OSO₂CH₃, OSO₂-Tolyl, OSO₂CF₃ oder OSO₂C₄F₉ darstellt, mit Olefinen der Formel **II,** 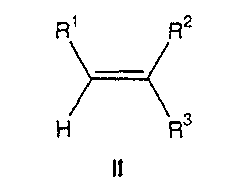 worin R¹, R² und R³ die gleiche Bedeutung wie bei Formel **III** haben, in Gegenwart von Palladiumkatalysatoren, **dadurch gekennzeichnet, daß** man als Katalysator eine Palladium-II-Verbindung der allgemeinen Formel PdXY oder deren CH₃CN-, C₆H₅CN- oder P(C₆H₅)₃-Komplexe, wobei X = Y = Cl, Br, I, RCO₂ (R = C₁ - C₂₂, CF₃, CCl₃, CH₂OCH₃, C₆H₅) oder RSO₃ ( R = C₁ - C₂₂, CF₃, C₄F₉, CCl₃, C₆H₅, *p*-CH₃C₆H₄) darstellt, in Gegenwart von Tetraarylphosphoniumsalzen Ar¹Ar²Ar³Ar⁴P⁺Z⁻, worin Ar¹, Ar², Ar³ und Ar⁴ gleiche oder unterschiedliche Arylreste bedeuten, typischerweise C₆H₅, *o*-, *m-, p*-CH₃-C₆H₄, *o-, m-, p*-Cl-C₆H₄ oder 1- oder 2-C₁₀H₇ und Z = Cl, Br, RCO₂ (R = C₁ - C₂₂, CF₃, CCl₃, C₆H₅) oder RSO₃ (R = C₁ - C₂₂, CF₃, C₄F₉, C₆H₅, *p*-CH₃C₆H₄), verwendet, und die Reaktion in Gegenwart eines dipolaren aprotischen Lösungsmittels und einer Base gfls. in Gegenwart eines Additivs bei Temperaturen von 60 °C bis 180 °C durchführt.

2. Verfahren nach Anspruch 1, **dadurch gekennzeichnet, daß** man als Katalysator eine Palladium-II-Verbindung der allgemeinen Formel PdXY oder deren CH₃CN-, C₆H₅CN- oder P(C₆H₅)₃-Komplexe, in monomerer oder oligomerer Form, wobei X = Cl, Br, I, RCO₂ [C₁ - C₂₂, CF₃, CCl₃, CH₂N(CH₃)₂, C₆H₅] oder RSO₃ (R = C₁ - C₂₂, CF₃, C₄F₉, CCl₃, C₆H₅, *p*-CH₃(C₆H₄) und Y = C₆H₅, *o*-, *m-, p*-CH₃C₆H₄, *o*-, *m*-, *p*-Cl-C₆H₄, *o*-, *m*-, *p*-CHOC₆H₄, *o*-, *m*-, *p*-CN-C₆H₄, *o*-, *m*-, *p*-NO₂-C₆H₄, *o-, m-, p*-PhCO-C₆H₄, *o-, m-, p*-F-C₆H₄, 1-C₁₀H₇ oder 2-C₁₀H₇, in Gegenwart von Tetraarylphosphoniumsalzen Ar¹Ar²Ar³Ar⁴P⁺Z⁻, worin Ar¹, Ar², Ar³ und Ar⁴ gleiche oder unterschiedliche Arylreste bedeuten, typischerweise C₆H₅, *o-, m-, p*-CH₃-C₆H₄, *o-, m-, p*-Cl-C₆H₄ oder 1- oder 2-C₁₀H₇ und Z = Cl, Br, RCO₂ (R = C₁ - C₂₂, CF₃, CCl₃, C₆H₅) oder RSO₃ (R = C₁ - C₂₂, CF₃, C₄F₉, C₆H₅, *p*-CH₃C₆H₄), verwendet, und die Reaktion in Gegenwart eines dipolaren aprotischen Lösungsmittels und einer Base ggfls. in Gegenwart eines Additivs bei Temperaturen von 60 °C bis 180 °C durchführt.

3. Verfahren nach Ansprüchen 1 - 2, **dadurch gekennzeichnet, daß** das Verhältnis von PdX₂ bzw. PdXY zu Phosphoniumsalz Ar¹Ar²Ar³Ar⁴P⁺Z⁻ 1: 1 bis 1 : 10 beträgt.

4. Verfahren nach Ansprüchen 1 - 3, **dadurch gekennzeichnet, daß** dipolare aprotische Lösungsmittel wie Dimethylsulfoxid, Dimethylformamid, Dimethylacetamid, 1,3-Dimethyl-3,4,5,6-tetrahydro-2-(1H)-pyrimidon oder N-Methylpyrrolidon verwendet werden.

5. Verfahren nach Ansprüchen 1 - 4, **dadurch gekennzeichnet, daß** als Base ein Amin, ein Alkali- oder Erdalkalimetallsalz einer Carbonsäure oder ein Alkali- oder Erdalkali-Carbonat oder -Bicarbonat eingesetzt wird.

6. Verfahren nach Ansprüchen 1 - 5, **dadurch gekennzeichnet, daß** die Reaktion im Temperaturbereich von 60 - 180 °C durchgeführt wird.

7. Verfahren nach Ansprüchen 1 - 6, **dadurch gekennzeichnet, daß** die Reaktion in Gegenwart eines selektivitätssteigernden stickstoffhaltigen Additivs durchgeführt wird.

8. Verfahren nach Anspruch 7, **dadurch gekennzeichnet, daß** eine stickstoffhaltige Carbonsäure oder deren Alkali- oder Erdalkalisalz als Additiv eingesetzt wird.

9. Verfahren nach Anspruch 7, **dadurch gekennzeichnet, daß** als Additiv ein stickstoffhaltiger Heterocyclus eingesetzt wird.

10. Verfahren nach Anspruch 8, **dadurch gekennzeichnet, daß** N,N-Dimethylglycin als Additiv verwendet wird.

11. Verfahren nach Anspruch 7, **dadurch gekennzeichnet, daß** als Additiv Pyridin eingesetzt wird.

12. Verfahren nach Ansprüchen 1 - 10, **dadurch gekennzeichnet, daß** die Base und das Additiv identisch sind.

13. Verfahren nach Ansprüchen 7 - 11, **dadurch gekennzeichnet, daß** das Verhältnis von PdX₂ bzw. PdXY zum Additiv 1 : 1 bis 1 : 100 beträgt.

## Claims

1. A process for the preparation of olefin-substituted aromatics or heteroaromatics of formula III 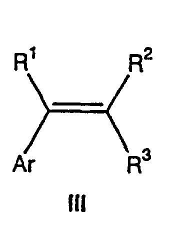 wherein Ar represents substituted or unsubstituted aryl or heteroaryl residues, and R¹, R² and R³ independently represent hydrogen, alkyl-(C₁-C₈), phenyl, 1- or 2-naphthyl, vinyl, O-alkyl-(C₁-C₈), O-phenyl, CN, CO₂H, CO₂-alkyl-(C₁-C₈), CO₂-phenyl, CONH₂, CONH-alkyl-(C₁-C₅), CON(alkyl)₂-(C₁-C₅), fluoro, chloro, PO(phenyl)₂, PO(alkyl)₂-(C₁-C₅), CO-phenyl, CO-alkyl-(C₁-C₅), NH-alkyl-(C₁-C₄), SO₃H, PO₃H, SO₃-alkyl-(C₁-C₄) or SO₂-alkyl-(C₁-C₄), or R¹ + R² = (CH₂)ₙ or R² + R³ = (CH₂)ₙ wherein n = 2-16, by reacting aromatics or heteroaromatics of formula I
ArX **I**
wherein Ar has the same meaning as in formula III and X represents chloro, bromo, OSO₂CH₃, OSO₂-tolyl, OSO₂CF₃ or OSO₂C₄F₉, with olefins of formula II 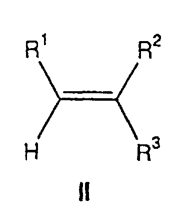 wherein R¹, R² and R³ have the same meanings as in formula III, in the presence of palladium catalysts, **characterized in that** a palladium(II) compound of general formula PdXY or its CH₃CN, C₆H₅CN or P(C₆H₅)₃ complexes, wherein X = Y = Cl, Br, I, RCO₂ (R = C₁-C₂₂, CF₃, CCl₃, CH₂OCH₃, C₆H₅) or RSO₃ (R = C₁-C₂₂, CF₃, C₄F₉, CCl₃, C₆H₅, p-CH₃C₆H₄), in the presence of tetraarylphosphonium salts Ar¹Ar²Ar³Ar⁴P⁺Z⁻, wherein Ar¹, Ar², Ar³ and Ar⁴ represent identical or different aryl residues, typically C₆H₅, o-, m-, p-CH₃-C₆H₄, o-, m-, p-Cl-C₆H₄ or 1- or 2-C₁₀H₇, and Z = Cl, Br, RCO₂ (R = C₁-C₂₂, CF₃, CCl₃, C₆H₅) or RSO₃ (R = C₁-C₂₂, CF₃, C₄F₉, C₆H₅, p-CH₃C₆H₄), is used as the catalyst, and the reaction is performed in the presence of a dipolar aprotic solvent and a base, optionally in the presence of an additive, at temperatures of from 60 °C to 180 °C.

2. The process according to claim 1, **characterized in that** a palladium(II) compound of general formula PdXY or its CH₃CN, C₆H₅CN or P(C₆H₅)₃ complexes in monomeric or oligomeric form, wherein X = Cl, Br, I, RCO₂ [R = C₁-C₂₂, CF₃, CCl₃, CH₂N(CH₃)₂, C₆H₅] or RSO₃ (R = C₁-C₂₂, CF₃, C₄F₉, CCl₃, C₆H₅, p-CH₃C₆H₄), and Y = C₆H₅, o-, m-, p-CH₃C₆H₄, o-, m-, p-Cl-C₆H₄, o-, m-, p-CHOC₆H₄, o-, m-, p-CN-C₆H₄, o-, m-, p-NO₂-C₆H₄, o-, m-, p-PhCO-C₆H₄, o-, m-, p-F-C₆H₄, 1-C₁₀H₇ or 2-C₁₀H₇, in the presence of tetraarylphosphonium salts Ar¹Ar²Ar³Ar⁴P⁺Z⁻, wherein Ar¹, Ar², Ar³ and Ar⁴ represent identical or different aryl residues, typically C₆H₅, o-, m-, p-CH₃-C₆H₄, o-, m-, p-Cl-C₆H₄ or 1- or 2-C₁₀H₇, and Z = Cl, Br, RCO₂ (R = C₁-C₂₂, CF₃, CCl₃, C₆H₅) or RSO₃ (R = C₁-C₂₂, CF₃, C₄F₉, C₆H₅, P-CH₃C₆H₄), is used as the catalyst, and the reaction is performed in the presence of a dipolar aprotic solvent and a base, optionally in the presence of an additive, at temperatures of from 60 °C to 180 °C.

3. The process according to claims 1-2, **characterized in that** the ratio of PdX₂ or PdXY to the phosphonium salt Ar¹Ar²Ar³Ar⁴P⁺Z⁻ is from 1:1 to 1:10.

4. The process according to claims 1-3, **characterized in that** dipolar aprotic solvents such as dimethyl sulfoxide, dimethylformamide, dimethyl acetamide, 1,3-dimethyl-3,4,5,6-tetrahydro-2(1H)-pyrimidone or N-methyl-pyrrolidone are used.

5. The process according to claims 1-4, **characterized in that** an amine, an alkali or alkaline earth metal salt of a carboxylic acid or an alkali or alkaline earth metal carbonate or bicarbonate is used as said base.

6. The process according to claims 1-5, **characterized in that** the reaction is performed in a temperature range of from 60 to 180 °C.

7. The process according to claims 1-6, **characterized in that** the reaction is performed in the presence of a selectivity-enhancing nitrogen-containing additive.

8. The process according to claim 7, **characterized in that** a nitrogen-containing carboxylic acid or its alkali or alkaline earth salt is used as said additive.

9. The process according to claim 7, **characterized in that** a nitrogen-containing heterocycle is used as said additive.

10. The process according to claim 8, **characterized in that** N,N-dimethyl-glycine is used as said additive.

11. The process according to claim 7, **characterized in that** pyridine is used as said additive.

12. The process according to claims 1 to 10, **characterized in that** said base and said additive are identical.

13. The process according to claims 7 to 11, **characterized in that** the ratio of PdX₂ or PdXY to the additive is from 1:1 to 1:100.

## Revendications

1. Procédé de synthèse de composés aromatiques ou hétéroaromatiques oléfiniques, de formule III, 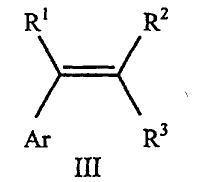 dans laquelle Ar représente des radicaux aryle ou hétéroaryle substitués ou non substitués, et R¹, R² et R³ représentent indépendamment l'un de l'autre un hydrogène, un alkyle en C₁ à C₈,un phényle, un 1- ou 2-naphtyle, un vinyle, un O-alkyle en C₁ à C₈, un O-phényle, CN, CO₂H, CO₂-alkyle en C₁ à C₈, CO₂-phényle, CONH₂, CONH-alkyle en C₁ à C₅, CON-(alkyle en C₁ à C₅)₂, un fluor, un chlore, PO(phényle)₂, PO(alkyle en C₁ à C₅)₂, CO-phényle, CO-(alkyle en C₁ à C₅), NH-alkyle en C₁ à C₄, SO₃H, PO₃H, SO₃-alkyle en C₁ à C₄ ou SO₂-alkyle en C₁ à C₄, ou R¹ + R² = (CH₂)ₙ ou R² + R³ = (CH₂)ₙ, n allant de 2 à 16, par réaction de composés aromatiques ou hétéroaromatiques de formule I
ArX I
dans laquelle Ar a la même signification que dans la formule III, et X représente un chlore, un brome, OSO₂CH₃, OSO₂-tolyle, OSO₂CF₃ ou OSO₂C₄F₉, avec des oléfines de formule II 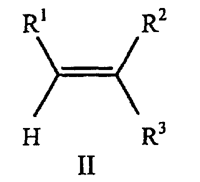 dans laquelle R¹, R² et R³ ont la même signification que dans la formule III, en présence de catalyseurs à base de palladium, **caractérisé en ce qu'**on prépare comme catalyseur un composé de palladium II de formule générale PdXY ou ses complexes avec CH₃CN, C₆H₅CN ou P(C₆H₅)₃ , où X = Y = Cl, I, Br, RCO₂ (R = C₁ à C₂₂, CF₃, CCl₃, CH₂OCH₃, C₆H₅) ou RSO₃ (R = C₁ à C₂₂), CF₃, C₄F₉, CCl₃, C₆H₅, *p*-CH₃C₆H₄), en présence de sels de tétraarylphosphonium Ar¹Ar²Ar³Ar⁴P⁺Z⁻, où Ar¹, Ar², Ar³ et Ar⁴ représentent des radicaux aryle identiques ou différents, typiquement C₆H₅, *o*-, *m-, p*-CH₃-C₆H₄, *o-, m-, p*-Cl-C₆H₄ ou 1- ou 2-C₁₀H₇ et Z = Cl, Br, RCO₂ (R = C₁ à C₂₂, CF₃, CCl₃, C₆H₅) ou RSO₃ (R = C₁ à C₂₂, CF₃, C₄F₉, C₆H₅, *p*-CH₃C₆H₄), et la réaction se déroule en présence d'un solvant aprotique dipolaire et d'une base, le cas échéant en présence d'un additif, à des températures allant de 60°C à 180°C.

2. Procédé selon la revendication 1, **caractérisé en ce qu'**on utilise comme catalyseur un composé de palladium II de formule générale PdXY ou ses complexes de CH₃CN, C₆H₅CN ou (P(C₆H₅), sous forme monomère ou oligomère, où X = Cl, Br, I, RCO₂ [en C₁ à C₂₂, CF₃, CCl₃, CH₂N(CH₃)₂, C₆H₅] ou RSO₃ (R = C₁ à C₂₂, CF₃, C₄F₉, CCl₃, C₆H₅, *p*-CH₃(C₆H₄) et Y = C₆H₅, *o-, m-, p*-CH₃C₆H₄, *o-, m-, p*-CHOC₆H₄, *o-, m-, p*-CN-C₆H₄, *o-, m-, p*-NO₂-C₆H₄, *o-, m-, p*-PhCO-C₆H₄, *o-, m-, p*-F-C₆H₄, 1-C₁₀H₇ ou 2-C₁₀H₇, en présence de sels de tétraarylphosphonium Ar¹Ar²Ar³Ar⁴P⁺Z⁻, où Ar¹, Ar², Ar³ et Ar⁴ représentent des radicaux aryle identiques ou différents, typiquement C₆H₅, *o-, m-, p*-CH₃-C₆H₄, *o-, m-, p*-Cl-C₆H₄ ou 1- ou 2-C₁₀H₇ et Z = Cl, Br, RCO₂ (R = C₁ à C₂₂, CF₃, CCl₃, C₆H₅) ou RSO₃ (R = C₁ à C₂₂, CF₃, C₄F₉, C₆H₅, *p*-CH₃C₆H₄), et **en ce qu'**on conduit la réaction en présence d'un solvant aprotique dipolaire et d'une base, le cas échéant en présence d'un additif, à des températures de 60°C à 180°C.

3. Procédé selon les revendications 1 et 2, **caractérisé en ce que** le rapport de PdX₂ ou selon les cas de PdXY au sel de phosphonium Ar¹Ar²Ar³Ar⁴P⁺Z⁻va de 1:1 à 1:10.

4. Procédé selon les revendications 1 à 3, **caractérisé en ce qu'**on utilise des solvants aprotiques dipolaires comme le diméthylsulfoxyde, le diméthylformamide, le diméthylacétamide, la 1,3-diméthyl-3,4,5,6-tétrahydro-2-(1H)-pyrimidone ou la N-méthylpyrrolidone.

5. Procédé selon les revendications 1 à 4, **caractérisé en ce qu'**on utilise comme base une amine, un sel de métal alcalin ou alcalino-terreux d'un acide carboxylique ou un carbonate ou bicarbonate de métal alcalin ou alcalino-terreux.

6. Procédé selon les revendications 1 à 5, **caractérisé en ce qu'**on conduit la réaction dans un intervalle de température allant de 60°C à 180°C.

7. Procédé selon les revendications 1 à 6, **caractérisé en ce qu'**on conduit la réaction en présence d'un additif contenant de l'azote et accroissant la sélectivité.

8. Procédé selon la revendication 7, **caractérisé en ce qu'**on utilise un acide carboxylique contenant de l'azote ou un de ses sels de métaux alcalins ou alcalino-terreux comme additif.

9. Procédé selon la revendication 7, **caractérisé en ce qu'**on utilise comme additif un hétérocycle contenant de l'azote.

10. Procédé selon la revendication 8, **caractérisé en ce qu'**on utilise la N,N-diméthylglycine comme additif.

11. Procédé selon la revendication 7, **caractérisé en ce qu'**on utilise comme additif la pyridine.

12. Procédé selon l'une des revendications 1 à 10, **caractérisé en ce que** la base et l'additif sont identiques.

13. Procédé selon l'une des revendications 7 à 11, **caractérisé en ce que** le rapport de PdX₂ ou selon les cas de PdXY à l'additif s'élève de 1:1 à 1:100.
